(19) Europäisches Patentamt / European Patent Office / Office européen des brevets

(11) **EP 1 726 272 B1**

(12) **EUROPÄISCHE PATENTSCHRIFT**

(45) Veröffentlichungstag und Bekanntmachung des Hinweises auf die Patenterteilung:
**08.07.2009 Patentblatt 2009/28**

(51) Int Cl.:
***A61F 2/16*** *(2006.01)*

(21) Anmeldenummer: **05011483.4**

(22) Anmeldetag: **27.05.2005**

(54) **Intraokularlinse**

Intraocular Lens

Lentille intraoculaire

(84) Benannte Vertragsstaaten:
**DE ES FR GB IT**

(43) Veröffentlichungstag der Anmeldung:
**29.11.2006 Patentblatt 2006/48**

(73) Patentinhaber: **WaveLight Laser Technologie AG**
**91058 Erlangen (DE)**

(72) Erfinder:
• **Donitzky, Christof**
**90542 Eckental (DE)**

• **Vogler, Klaus, Dr.**
**90542 Eckental (DE)**

(74) Vertreter: **von Hellfeld, Axel**
**Wuesthoff & Wuesthoff**
**Patent- und Rechtsanwälte**
**Schweigerstrasse 2**
**81541 München (DE)**

(56) Entgegenhaltungen:
**US-A- 4 373 218        US-A- 4 787 903**
**US-A- 5 171 266        US-A- 5 443 506**
**US-A1- 2004 169 932    US-B1- 6 730 123**

**Beschreibung**

Die Erfindung betrifft eine Intraokularlinse, die geeignet ist, in ein menschliches Auge eingesetzt zu werden.

In der Ophthalmologie sind Intraokularlinsen weithin bekannt, zum Beispiel werden bei einer Eintrübung der ursprünglich klaren Augenlinse ("grauer Star" bzw. Katarakt) Intraokularlinsen implantiert.

Ein besonders Problem in der Ophthalmologie ist die altersbedingte Weitsichtigkeit (Presbyopie), vgl. z.B. den Aufsatz "Intraokularlinsen", in DER AUGENSPIEGEL, 7-8/2002: 28-34. Der altersbedingte Verlust der Elastizität der natürlichen Augenlinse verhindert deren Brechkraftanpassung und damit eine Akkommodation des Auges auf im Nahbereich vor dem Auge liegende Objekte und deren scharfe Abbildung auf der Netzhaut. Zwar lässt sich der Verlust der Akkommodationsfähigkeit durch eine Lesebrille korrigieren, jedoch ist dies mit dem bekannten Aufwand und Unbequemlichkeiten verbunden.

Der Stand der Technik kennt vielfältige Bemühungen, das Problem der Presbyopie zu lösen:

So gab es den Versuch, künstliche Intraokularlinsen (IOL) durch Wirkung des Ziliarmuskels des Auges zu verschieben (vgl. den oben zitierten Aufsatz in "Der Augenspiegel"). Jedoch ergab sich dabei eine nur ungenügende Verschiebung der IOL in Axialrichtung (üblicherweise als z-Achse bezeichnet) und somit auch nur eine ungenügende Brennpunktverschiebung.

Es wurde auch versucht, eine eingesetzte IOL durch den Ziliarmuskel mechanisch zu deformieren, wobei allerdings der Erfolg noch nicht überzeugend nachgewiesen werden konnte.

Ein anderer Ansatz versucht die Wiederherstellung der Elastizität der natürlichen Augenlinse durch radiale Schnitte in der kristallinen Augenlinse mit einem nichtinvasiven FS-Laser, vgl. O. Kermani in "Neues aus Wissenschaft und Forschung", J. Refract Surgery: 2004; 20:651-658. Allerdings ändert sich dabei die Elastizität kaum und es besteht die Gefahr eines induzierten Kataraktes.

Weiterhin wurden multifokale IOL implantiert, allerdings mit den bekannten Schwierigkeiten, insbesondere störenden Doppelbildern und Unschärfe, da zumindest zwei Brennweiten mit unterschiedlichen Abbildungseigenschaften simultane Bilder auf der Netzhaut erzeugen, vgl. "Neue Mulfifokallinsen, individuelle Lösungen und postoperative Funktionalität", in Der Augenspiegel, 09/2004:42-47.

Ein weiterer Versuch der Behebung der Presbyopie ist die Erzeugung einer Monovision durch refraktive Laserchirurgie der Hornhaut. Dabei wird ein Auge auf den Nahpunkt korrigiert und das andere Auge auf den Fempunkt eingestellt belassen oder dahingehend korrigiert, letzteres ist dann meist das sogenannte Führungsauge. Dabei liefern aber beide Auge unterschiedliche Informationen, die vom Gehirn verarbeitet und vom Patienten angenommen werden müssen.

Die Druckschrift US 4,373,218 betrifft einen expandierbaren Sack, in dem ein Fluid enthalten ist. Ein Ventil ist vorgesehen, um mehr oder weniger Flüssigkeit in den Sack zu lassen, um die Brechungseigenschaften der Linse zu verändern. In einer Ausführungsform wird eine Spannung über flüssig-kristallines Material in dem Sack gelegt, um so dessen Brechungsindex zu ändern. Dabei wird der Brechungsindex des Materials selbst geändert, nicht jedoch die Form des Sacks.

Die Druckschrift 5,171,266 betrifft eine Unse mit einem darum angeordneten Haltering. In dem Haltering sind Spulen angeordnet. Bei Anlegen einer elektrischen Spannung an die Spulen wird ein magnetisches Feld erzeugt, welches den Haltering ausdehnt bzw. zusammenzieht, wodurch sich die Form der Linse ändert.

Die vorliegende Erfindung geht einen anderen Weg zur Wiederherstellung der Akkommodationsfähigkeit des menschlichen Auges.

Hierzu lehrt die Erfindung eine Intraokularlinse mit den Merkmalen des Anspruchs 1.

Die Brechkraft der Intraokularlinse ist durch Anlegen elektrischer Spannungen veränderbar. Gegenstand der Erfindung ist also eine künstliche Intraokularlinse, die geeignet ist, in das menschliche Auge eingesetzt zu werden und die so gestaltet ist, dass ihre Brechkraft durch elektrische Spannungen oder Felder veränderbar ist, sodass die Akkommodationsfähigkeit des Auges wiederhergestellt ist.

In anderen Anwendungsbereichen sind sogenannten Flüsslgkeitslinsen seit einiger Zeit bekannt. Bei ihnen wird durch das Anlegen von elektrischen Spannungen eine Formänderung der Flüssigkeitslinse und damit eine Änderung von deren Brennweite bewirkt. Der zugrundeliegende physikalische Effekt wird auch mit "Elektro-Wetting" bezeichnet. Der Begriff "Flüssigkeitslinse" erfasst hier auch andere verformbare, insbesondere liquidartige Materialien. Durch geeignet gestaltete Elektroden, ausgewählte Viskositäten und Dichten des Linsenmaterials und eine geeignete Dimensionierung der Linse sowie Vorkehrungen zur Sicherung der Lagestabilität der Linse lässt sich eine sehr hohe Dynamik erreichen, d.h. eine sehr schnelle Änderung der Linsenform, zum Beispiel ist z.Z. schon eine Frequenz der Brennweitenänderung im Bereich von etwa 2 kHz möglich. Diese Dynamik ist wesentlich besser als es mit einem mechanischen Zoom möglich wäre. Es lassen sich solche durch elektrische Spannungen betätigbare Flüssigkeitslinsen mit Durchmessern im Bereich von 3 bis 6 mm herstellen. Zum Stand der Technik wird insoweit auf folgende Veröffentlichungen verwiesen:

Kuiper S., Hendriks BHW.: Variable-focus Liquid Lens for Miniature Cameras, Appl Phys Lett 2004; 85:1128-1130;

Hecht E., Opitics-Second Edition. Addison-Wesley Publishing Company, Chapter 5: Geometrical Optics-Paraxial Theory: p- 138;

Krupenkin T., Yang S., Mach P.: Tunable Liquid Microlens, Appl Phys Lett 2003; 82:316-318 und

Berge B., Peseux J.: Variable focal lens controlled by an external voltage: An application of electrowetting. Eur Phys J 2000; E3: 159-163.

Die erfindungsgemäße Intraokular-Flüssigkeitslinse ist so dimensioniert und gestaltet, dass sie in den Kapselsack eines Auges einsetzbar ist. Auch kann die Intraokular-Flüssigkeitslinse so dimensioniert und gestaltet sein, dass sie in einen Sulkus einsetzbar ist.

Weiterhin weist die derart einsetzbare IOL Mittel auf, damit durch Anlegen einer elektrischen Spannung die mechanische Oberflächenspannung der Linse zur Änderung ihrer Brennweite veränderbar ist.

Gemäß einer bevorzugten Ausgestaltung ist die Linse so dimensioniert und sind die Mittel zum Anlegen einer elektrischen Spannung so gestaltet, dass die elektrische Spannung eine sphäro-symmetrische Deformation der Linse bewirkt. Dabei ist in der Linse eine Radialsymmetrie vorgegeben. Auch kann die Linse so dimensioniert und gestaltet sein, dass die elektrischen Spannungen einen Astigmatismus sowie Aberrationen, insbesondere sphärische Aberrationen, also Augenfehler höherer Ordnung kompensieren.

Das sogenannte "Elektro-Wetting" verändert die mechanische Oberflächenspannung der Flüssigkeit bzw. des liquidartigen oder anders geeigneten Materials, wobei die Linse so gestaltet und/oder so äußeren Kräften ausgesetzt ist, dass sich aufgrund der veränderten Oberflächenspannung ihre Form gegenüber einem Zustand ohne Spannung oder bei anderen Spannungen ändert.

Gemäß besonderen Ausgestaltungen der erfindungsgemäßen IOL können diese ganz oder teilweise von einer Membran bedeckt sein und gegebenenfalls zusätzliche Fixationselemente aufweisen, wie letzteres bei herkömmlichen Intraokularlinsen bekannt ist. Fixationselemente können an der Linse vorgesehen sein, um diese im Auge in der gewünschten Weise positionieren zu können.

Die Formgebung der erfindungsgemäßen IOL in einem Kapselsack im spannungsfreien Zustand kann durch das Einbringen ergänzender hydrophober Oberflächen und einer damit einhergehenden Veränderung der Oberflächenspannung in gewünschter Weise beeinflusst werden.

Gemäß besonderen Ausgestaltungen der Erfindung ist die intraokulare Linse mit Elektroden versehen zum Anlegen der genannten elektrischen Spannungen. Dabei sind die Elektroden zumindest teilweise transparent. Auch sind gemäß einer bevorzugten Ausgestaltung der Erfindung die Elektroden in geeigneten Abständen quasi rundum um die IOL angeordnet.

Die für die Steuerung der erfindungsgemäßen IOLs erforderlichen elektrischen Spannungen können in verschiedener Weise gewonnen und eingesetzt werden. Zum Beispiel ist es möglich, stark miniaturisierte elektronischen Komponenten einzusetzen. Es werden heute schon solche Komponenten in das menschliche Auge implantiert, vgl. z.B. "Eine Prothese zum Sehen", in AUGENLICHT 2003; 1:26-27. Der Stand der Technik kennt einige Mittel, Energie und Signale in das humane Auge einzubringen, vgl. H.G. Sachs und V.P. Gabel in Graefe's Arch. Clin. Exp. Ophthalmol. (2004) 242:717-723; Aufsatz "Die Implantation von Sehprothesen bei progressiven Netzhautdystrophien" von P. Walter, in der AUGENSPIEGEL, 11/2004, S. 32; T. Laube, C. Brockmann, R. Buß, C. Lau, K. Höck, N. Stawski, T. Stieglitz, H.A. Richter und H. Schilling in Graefe's Arch. Clin. Exp. Ophthalmol. (2004) 242:661-667; und T. Stieglitz, R. Keller, H. Beutel und J.U. Meyer, "Microsystem Integration Techniques for Intraocular Vision Prostheses Using Flexible Polyimide Foils".

Andererseits ist auch eine physiologische Bereitstellung der elektrischen Spannungen möglich: Zum Beispiel kann die für die Akkommodation benötigte Spannung direkt aus einer Bewegung des Auges abgeleitet werden, insbesondere durch eine triboelektrische Spannungserzeugung, also eine Spannungserzeugung durch den Reibungseffekt. Mit Hilfe eines implantierten Mikrochips kann diese Spannung auf das erforderliche Maß verstärkt und an die geeignet gestalteten Elektroden der Linse angelegt werden, um deren Oberflächenkrümmung einzustellen. So ist zum Beispiel mit dem natürlichen Akkommodationsvorgang eine Betätigung des Ziliarmuskels verbunden. Der Ziliarmuskel bewirkt beim jungen, voll funktionsfähigen Auge eine Verformung der Linse zur Akkommodation. Eine besondere Ausgestaltung der vorliegenden Erfindung stellt Mittel bereit, mit denen aus dieser Wirkung des Ziliarmuskels eine elektrische Spannung abgeleitet wird, die dann, falls erforderlich, ausreichend verstärkt an die genannten Elektroden der Linse angelegt wird, um die gewünschte Akkommodation in Abhängigkeit von der natürlichen Bewegung des Ziliarmuskels zu bewirken.

Durch besondere Anordnung der Elektroden können auch Abbildungsfehler höherer Ordnung sowie astigmatische Abbildungsfehler überwunden werden, vgl. EP 1 091 758 B1 und US 6,369,954 B1.

Nachfolgend werden Ausführungsbeispiele der Erfindung anhand der Zeichnung näher beschrieben. Es zeigt:

Figur 1    einen schematischen Schnitt durch ein Auge, in das eine erfindungsgemäße Intraokularlinse eingebaut ist, wobei die Linse nicht akkommodiert ist;

Figur 2    eine Darstellung entsprechend Figur 1, wobei die Linse akkommodiert ist;

Figur 3    eine weitere Ausgestaltung einer IOL mit einem Mikrochip zur Spannungssteuerung, wiederum in einem in das Auge einbauten Zustand zur Illustration der Wirkungsweise.

Grundsätzlich kann die Brechkraft- bzw. Brennweitenänderung einer Linse durch die einfache Linsengleichung dargestellt werden, wobei die Krümmungsradien der Linse deren Brennweite bestimmen (vgl. z.B. das oben zitierte Lehrbuch von E. Hecht):

$$\frac{1}{f} = (n-1) \cdot \left( \frac{1}{R_1} - \frac{1}{R_2} \right)$$

$R_1$– Krümmungsradien der Linsenoberfläche    (1)

n- Brechzahl

Wird eine erfindungsgemäße Intraokularlinse (IOL) in den zuvor entsprechend präparierten Kapselsack des Auges eingesetzt, ergibt sich im wesentlichen eine zentrierte Position der Linse im optischen Strahlengang des Auges. Es ist auch möglich, die Flüssigkeitslinse mit einer eigenen Membran zu umgeben und mit zusätzlichen Fixationselementen zu versehen, um ihre zentrale Lage auf der optischen Achse des Auges zu gewährleisten. Diese Technik ist als solches von herkömmlichen IOLs bekannt.

Die Änderung der Brechkraft D einer Flüssigkeitslinse kann durch folgende Gleichung beschrieben werden:

$$D = D_0 + K \frac{U^2}{d}$$

K=Materialkonstante    (2)

d=Durchmesser der Linse

U=Spannung

Somit ist die Brechkraftänderung einer Flüssigkeitslinse grundsätzlich quadratisch abhängig von der angelegten Spannung und umgekehrt proportional zum Durchmesser der Linse. Deshalb werden bei kleinen Linsendurchmessern, wie hier, und geeigneten dielektrischen Isolationsschichten nur relativ geringe Spannungen benötigt, um merkliche Brechkraftänderungen zu erzielen (vgl. den oben zitierten Aufsatz von T. Krupenkin et al.).

Im menschlichen Auge vollbringt die Augenlinse einen Akkommodationshub von ca. 10 dpt auf etwa 14 dpt in Abhängigkeit des Alters.

Nimmt man zur Zeit zur Verfügung stehende elektro-optische Konstanten für das Flüssiglinsenmaterial an (vgl. die obigen Arbeiten von S. Kuiper et al., und T. Krupenkin et al.) so können mit den heute bekannten Materialien bereits mit Spannungen im Bereich von U=20-30 V solche Brechkraftänderungen erreicht werden.

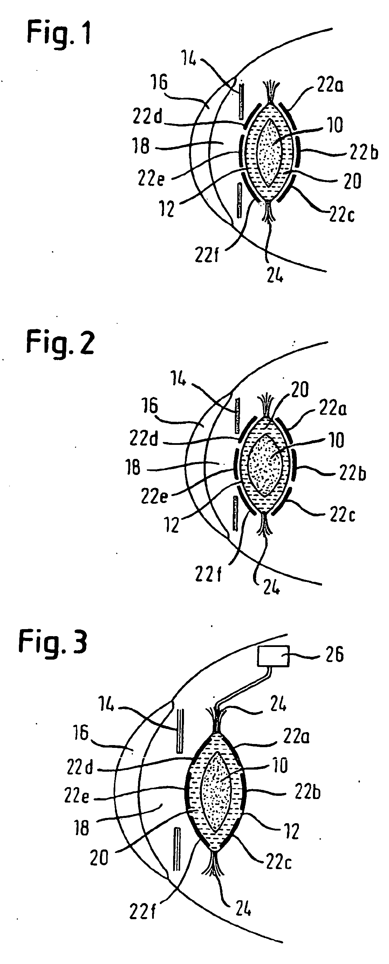

Figur 1 zeigt eine Intraokularlinse 10, die in einen Kapselsack 12 eines menschlichen Auges eingesetzt ist. Dargestellt sind weiterhin die Iris 14, die Kornea 16 und die Vorderkammer 18 des Auges.

Die Intraokularlinse 10 aus einem Flüssigmaterial der oben beschriebenen Art ist von einer isolierenden Flüssigkeit 20 umgeben.

Gemäß den Figuren sind Elektroden 22a, 22b, 22c, 22d, 22e, 22f entweder um den Kapselsack 12 herum (Figuren 1 und 2) oder innenseitig am Kapselsack (Figur 3) angeordnet. Auch können die Elektroden in der Äquatorebene des Kapselsacks angeordnet werden. Andererseits kann eine komplett gekapselte Intraokularlinse mit innenliegenden Elektroden verwendet werden.

Der natürliche Ziliarmuskel 24 greift senkrecht zur optischen Achse des Systems "Auge" am Kapselsack an.

Figur 1 zeigt schematisch den nicht akkommodierten Zustand der Intraokularlinse 20, also den Zustand, in dem an den Elektroden keine Spannung anliegt und somit in der obigen Gleichung (2) $D=D_0$ gilt. Die senkrecht zur optischen Achse wirkende Streckkraft bzw. Stauchkraft F ist ebenfalls gleich null F=0.

Figur 2 zeigt einen Zustand, in dem eine Spannung U an die Elektroden 22 angelegt ist. Es gilt $D=D_0+KU^2$. Die Intraokularlinse wird elektrostriktiv kontrahiert (akkommodiert) und es wirkt eine Kraft senkrecht zur optischen Achse, die verschieden von null ist, also die Formänderung in der gewünschten Weise bewirkt, sodass der Krümmungsradius

4

der Grenzfläche der Linse 10 stark variiert und somit die Brechkraft vergrößert wird.

Figur 3 zeigt ein abgewandeltes Ausführungsbeispiel, bei dem ein Mikrochip 26 zum System gehört, der ebenfalls im eingebauten Zustand in Figur 3 dargestellt ist. Einander funktionsgleiche oder funktionsähnliche Bauteile sind in den Figuren mit gleichen Bezugzeichen versehen.

Beim Ausführungsbeispiel gemäß Figur 3 wird eine triboelektrische Spannungserzeugung ausgenutzt. Wie oben beschrieben, ist mit dem natürlichen Akkommodationsvorgang eine Kraft verknüpft, zum Beispiel diejenige, die durch den Ziliarmuskel auf die natürliche Augenlinse ausgeübt wird. Beim Ausführungsbeispiel gemäß Figur 3 wird aus dieser Kraftwirkung eine Spannung abgeleitet und ausreichend verstärkt, um an die Elektroden 22a, ...., 22f angelegt zu werden und so eine entsprechende Deformation der Grenzfläche und damit deren Akkommodation zu bewirken.

Die triboelektrische Spannungserzeugung ist ähnlich dem piezoelektrischen Effekt beschreibbar, dort als Ladungserzeugung durch eine Kraft, hier durch eine bewegungsauslösende Kraft und eine Ladungstrennung, vgl. Benz W., Heinks P., Starke L.: Tabellenbuch Elektronik für Industrie-Elektroniker und Kommunikationselektroniker. Kohl + Noltemeyer Verlag: S.87. Es gilt:

$$\Delta e / e \sim e_{12} \frac{F_{12}}{A}$$

$e_{12}$ – Elastizitätsmodul $\qquad$ (3)

$\Delta e/e$- relative Längenänderung

$F_{12}$ - Kraftkomponente

$A$ - Fläche

$$U = \frac{Q}{C} = \frac{1}{C} S_{12} F_{12}$$

$S_{12}$ - piezoelektrisches Modul $\qquad$ (4)

$C$ - Kapazität

Diese triboelektrische Spannung wird über den implantierten Mikrochip 26 verstärkt und dieser Chip ist über Leitungen (nicht gezeigt) mit den einzelnen Elektroden 22a, ...., 22f verbunden und steuert diese so, dass die gewünschte Akkommodation erreicht wird.

**Patentansprüche**

1. Intraokularlinse (10), mit Mitteln (22a, 22b, 22c, 22d, 22e, 22f) zum Anlegen einer elektrischen Spannung, **dadurch gekennzeichnet, dass** die Mittel dazu eingerichtet sind, die elektrische Spannung an die Intraokularlinse (10) anzulegen und die Form der Intraokularlinse (10) durch Anlegen der elektrischen Spannung und eine **dadurch** geänderte mechanische Oberflächenspannung der Intraokularlinse (10) zur Änderung von deren Brechkraft zu verändern, wobei die Intraokularlinse (10) eine Flüssigkeitslinse umfasst.

2. Intraokularlinse nach Anspruch 1, die so gestaltet ist, dass sie In den Kapselsack (12) eines Auges oder einen Sulcus einsetzbar ist.

3. Intraokutarlinse nach einem der vorhergehenden Ansprüche, **dadurch gekennzeichnet, dass** die elektrische Spannung eine sphäro-symmetrische Deformation der Unse (10) bewirkt.

4. Intraokularlinse nach einem der Ansprüche 1 oder 2, **dadurch gekennzeichnet, dass** die elektrische Spannung eine Deformation der Unse (10) zur Kompensation eines Astigmatismus oder zur Kompensation von Aberrationen höherer Ordnung erzeugt.

5. Intraokularlinse nach einem der vorhergehenden Ansprüche, **dadurch gekennzeichnet dass** die Unse (10) von einer Membran bedeckt ist.

**6.** Intraokularlinse nach einem der vorhergehenden Ansprüche mit Fixationselementen zur Positionierung im Auge.

**7.** Intraokularlinse nach einem der vorhergehenden Ansprüche, **dadurch gekennzeichnet, dass** die Linse (10) mit Elektroden (22a, 22b, 22c, 22d, 22e, 22f) zum Anlegen elektrischer Spannungen versehen ist.

**8.** Intraokularlinse nach Anspruch 7, **dadurch gekennzeichnet, dass** die Elektroden (22a, 22b, 22c, 22d, 22e, 22f) zumindest teilweise transparent sind.

**9.** Intraokularlinse nach einem der vorhergehenden Ansprüche, **gekennzeichnet durch** einen Mikrochip (26) zum Verarbeiten und Verstärken triboelektrischer Spannungen.

**Claims**

**1.** Intraocular lens (10) comprising means (22a, 22b, 22c, 22d, 22e, 22f) for applying an electrical voltage, **characterized in that**
the means are adapted to apply the electrical voltage at the intraocular lens (10) and to change the form of the intraocular lens (10) by applying the electrical voltage and a thereby changed mechanical surface tension of the intraocular lens (10) for changing of its refraction power, whereas the intraocular lens (10) comprises a liquid lens.

**2.** Intraocular lens according to claim 1, which is formed in that it is insertable in a capsular bag of an eye or a sulcus.

**3.** Intraocular lens according to one of the preceding claims, **characterized in that** the electrical voltage causes a sphere-symmetrical deformation of the lens (10).

**4.** Intraocular lens according to one of claims 1 or 2, **characterized in that** the electrical voltage causes a deformation of the lens (10) for compensation of an astigmatism or for compensation of aberration of higher order.

**5.** Intraocular lens according to one of the preceding claims, **characterized in that** the lens (10) is covered by a membrane.

**6.** Intraocular lens according to one of the preceding claims having fixation elements for positioning in an eye.

**7.** Intraocular lens according to one of the preceding claims, **characterized in that** the lens (10) is provided with electrodes (22a, 22b, 22c, 22d, 22e, 22f) for applying electrical voltages.

**8.** Intraocular lens according to claim 7, **characterized in that** the electrodes (22a, 22b, 22c, 22d, 22e, 22f) are at least partially transparent.

**9.** Intraocular lens according to one of the preceding claims, **characterized by** a micro chip (26) for processing and amplifying of triboelectrical voltages.

**Revendications**

**1.** Lentille intraoculaire (10), comprenant des moyens (22a, 22b, 22c, 22d, 22e, 22f) pour l'application d'une tension électrique, **caractérisée en ce que** les moyens sont aménagés pour appliquer la tension électrique sur la lentille intraoculaire (10) et modifier la forme de la lentille intraoculaire (10) par l'application de la tension électrique et une tension de surface mécanique ainsi modifiée de la lentille intraoculaire (10) pour la modification de son pouvoir réfringent, la lentille intraoculaire (10) comportant une lentille liquide.

**2.** Lentille intraoculaire selon la revendication 1, qui est conçue de telle sorte qu'elle peut être insérée dans le sac capsulaire (12) d'un oeil ou dans un sillon.

**3.** Lentille intraoculaire selon l'une des revendications précédentes, **caractérisée en ce que** la tension électrique provoque une déformation sphéro-symétrique de la lentille (10).

**4.** Lentille intraoculaire selon l'une des revendications 1 ou 2, **caractérisée en ce que** la tension électrique génère une déformation de la lentille (10) pour la compensation d'un astigmatisme ou pour la compensation d'aberrations d'ordre supérieur.

**5.** Lentille intraoculaire selon l'une des revendications précédentes, **caractérisée en ce que** la lentille (10) est recouverte d'une membrane.

**6.** Lentille intraoculaire selon l'une des revendications précédentes, comprenant des éléments de fixation pour le positionnement dans l'oeil.

**7.** Lentille intraoculaire selon l'une des revendications précédentes, **caractérisée en ce que** la lentille (10) est dotée d'électrodes (22a, 22b, 22c, 22d, 22e, 22f) pour l'application de tensions électriques.

**8.** Lentille intraoculaire selon la revendication 7, **caractérisé en ce que** les électrodes (22a, 22b, 22c, 22d, 22e, 22f) sont au moins en partie transparentes.

**9.** Lentille intraoculaire selon l'une des revendications précédentes, **caractérisée par** une micropuce (26) pour le traitement et l'amplification de tensions triboélectriques.

## Fig. 1

## Fig. 2

## Fig. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4373218 A
- EP 1091758 B1
- US 6369954 B1

**In der Beschreibung aufgeführte Nicht-Patentliteratur**

- **O. Kermanl.** Neues aus Wissenschaft und Forschung. *J. Refract Surgery,* 2004, vol. 20, 651-658
- **KUIPER S. ; HENDRIKS BHW.** Variable-focus Liquid Lens for Miniature Cameras. *Appl Phys Lett,* 2004, vol. 85, 1128-1130
- **HECHT E.** Geometrical Optics-Paraxial Theory. Addison-Wesley Publishing Company, 138
- **KRUPENKIN T. ; YANG S. ; MACH P.** Tunable Liquid Microlens. *Appl Phys Lett,* 2003, vol. 82, 316-318
- **BERGE B. ; PESEUX J.** Variable focal lens controlled by an external voltage: An application of electrowetting. *Eur Phys J,* 2000, vol. E3, 159-163
- Eine Prothese zum Sehen. *AUGENLICHT,* 2003, vol. 1, 26-27
- **H.G. SACHS ; V.P. GABEL.** *Graefe's Arch. Clin. Exp. Ophthalmol.,* 2004, vol. 242, 717-723
- **P. WALTER.** Die Implantation von Sehprothesen bei progressiven Netzhautdystrophien. *AUGEN-SPIEGEL,* November 2004, 32
- **T. LAUBE ; C. BROCKMANN ; R. BUß ; C. LAU ; K. HÖCK ; N. STAWSKI ; T. STIEGLITZ ; H.A. RICHTER ; H. SCHILLING.** *Graefe's Arch. Clin. Exp. Ophthalmol.,* 2004, vol. 242, 661-667
- **T. STIEGLITZ ; R. KELLER ; H. BEUTEL ; J.U. MEYER.** *Microsystem Integration Techniques for Intraocular Vision Prostheses Using Flexible Polyimide Foils*